# EUROPEAN PATENT SPECIFICATION

(11) **EP 4 287 991 B1**
(45) Date of publication and mention of the grant of the patent: **01.04.2026**
(21) Application number: 22701662.3
(22) Date of filing: 02.02.2022
(51) Int. Cl.: A61F 2/24, A61B 5/00

(54) **REPLACEMENT HEART VALVE PROSTHESIS WITH A PREDEFINED PASSAGE**
ERSATZHERZKLAPPENPROTHESE MIT VORDEFINIERTEM DURCHGANG
PROTHÈSE DE VALVULE CARDIAQUE DE REMPLACEMENT DOTÉE D'UN PASSAGE PRÉDÉFINI

(30) Priority: 03.02.2021 EP 21154930
(43) Date of publication of application: 13.12.2023
(73) Proprietor: Tricares SAS, 75001 Paris (FR)
(72) Inventor: STRAUBINGER, Helmut, 85609 Aschheim (DE)
(74) Representative: Hoefer & Partner Patentanwälte mbB
(86) International application number: PCT/EP2022/052398
(87) International publication number: WO 2022/167443

(56) References cited:
- WO-A1-2021/080782
- US-A1- 2019 060 062

## Description

The present disclosure concerns a replacement heart valve prosthesis with a predefined passage, a system comprising same and a method for implantation of said system and a method for introducing an accessory means through the predefined passage of a replacement heart valve prosthesis or a method for implanting a pacemaker lead or electrode or any other cable into a heart.

### BACKGROUND OF THE DISCLOSURE

The replacement heart valve prosthesis technology is well advanced and it is possible to provide patients in a minimally invasive manner with a number of replacement heart valve prostheses, e.g. in the aortic, mitral, pulmonary or tricuspid heart valve.

It is essential when implanting such prostheses to achieve a good sealing and to avoid paravalvular leakage.

Many patients prone for a replacement heart valve prosthesis procedure have already a pacemaker implanted. The pacemaker leads are usually positioned so that it passes through a natural heart valve and is touching or fixed in the area of the apex of the heart to trigger a regular heart beating.

The implantation of a replacement heart valve prosthesis in a patient already carrying a pacemaker implies the issue that the pacemaker lead will be pushed to the sidewall of the natural valve and be fixed there by the replacement heart valve prosthesis after its expansion. As an disadvantageous effect the problem arises that paravalvular leakage will occur in the area of the pacemaker lead positioned between the natural heart valve wall and the replacement heart valve prosthesis.

Moreover, in many cases the fact that a patient is carrying a pacemaker is an exclusion criteria for a replacement heart valve prosthesis implantation. Hence, a number of patients who need a replacement heart valve prosthesis will not receive such lifesaving treatment.

It was thus an object underlying the present application to provide a means to reduce or essentially avoid issues relating to the implantation of a pacemaker and a replacement heart valve prosthesis, or at least to improve or avoid the disadvantages of a replacement heart valve prosthesis treatment linked to the existence or need of a pacemaker.

It was thus another object underlying the present application to provide a means to reduce or essentially avoid paravalvular leakage linked to the concomitant implantation of an accessory means like a pacemaker or defibrillator or any other device or electrode or probe wherein said accessory means must passage through or passage sideways of the replacement heart valve prosthesis. US 2019060062 A1 discloses a transcatheter prosthesis for intervascular delivery, is has a fixation unit extending outwardly from outer surface of frame.

WO2021/080782 A1 refers to a delivery system for replacing heart valves, it has a deflection mechanism which is configured to deflect portion that is positioned proximal of bend portion to deflect bend portion towards second direction that is opposed to first direction.

### BRIEF SUMMARY OF THE DISCLOSURE

In one aspect the disclosure relates to a replacement heart valve prosthesis comprising a sealing material wherein the sealing material contains at least one predefined passage suitable for an accessory means like a catheter, a probe or an electrode or a pacemaker or defibrillator lead.

In another aspect the disclosure relates to a system comprising a replacement heart valve prosthesis as described herein and an accessory means like a catheter, a probe, an electrode, a defibrillator or a pacemaker lead.

In another aspect the disclosure relates to a method for introducing an accessory means like a defibrillator or/and a pacemaker lead in a patient carrying a replacement heart valve prosthesis as described herein wherein the predefined passage can be visualized and the accessory means is introduced into and pushed through the predefined passage(s) and positioned in its/Their final target position(s).

### DETAILED DESCRIPTION OF THE FIGURES

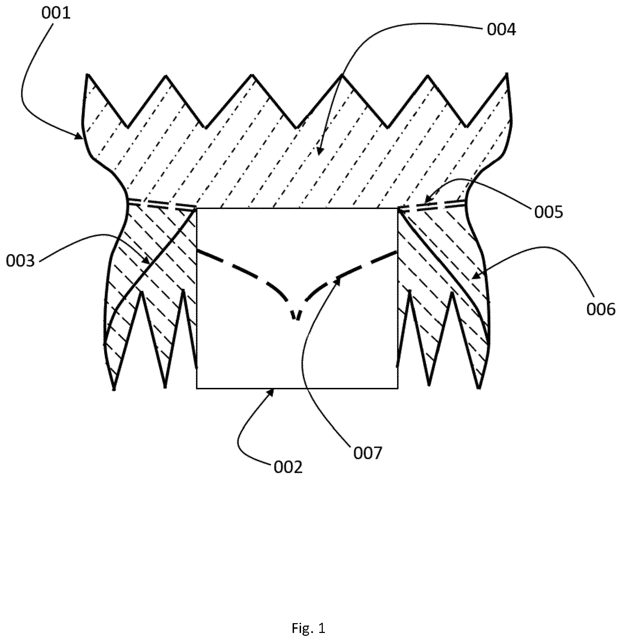

Fig. 1 depicts a replacement heart valve prosthesis comprising an outer stent 001 and an inner stent 002 including connecting arms 003, an atrial sealing 004, a sealing ring 005 and a ventricular sealing 006 according to the prior art.

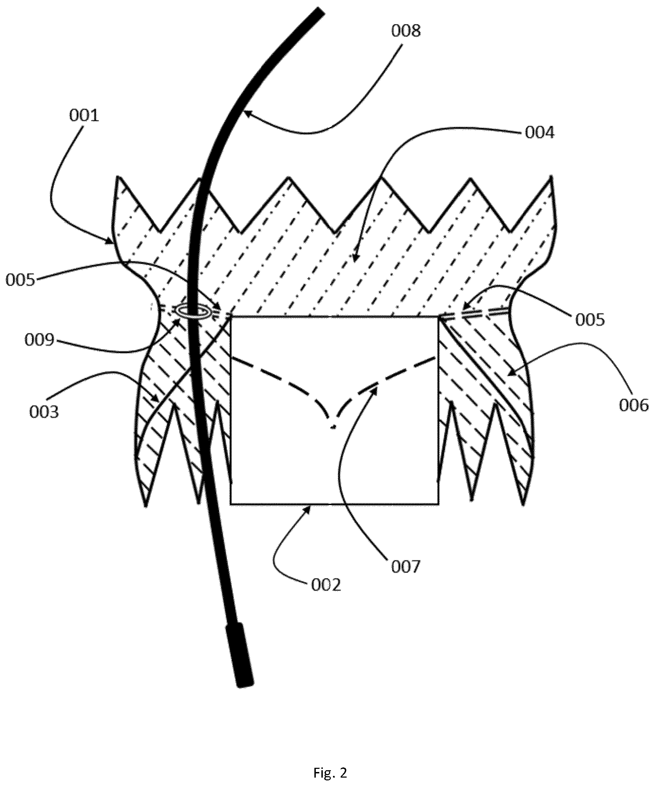

Fig. 2 in addition to the features of Fig. 1 illustrates a pacemaker lead 008 through lead gateway 009 wherein the lead gateway is positioned within the sealing ring 005.
Fig. 3 is a top view of the replacement heart valve prosthesis of Fig. 2 wherein a lead gateway 009 is positioned in the sealing ring 005 between the outer stent 001 and inner stent 002. The lead gateway 009 is composed of a suture line 012 within which an opening is created. For sealing purposes the sealing ring 005 within the lead gateway may be opened by way of a puncture or a cut to create a passage way for the lead 008.

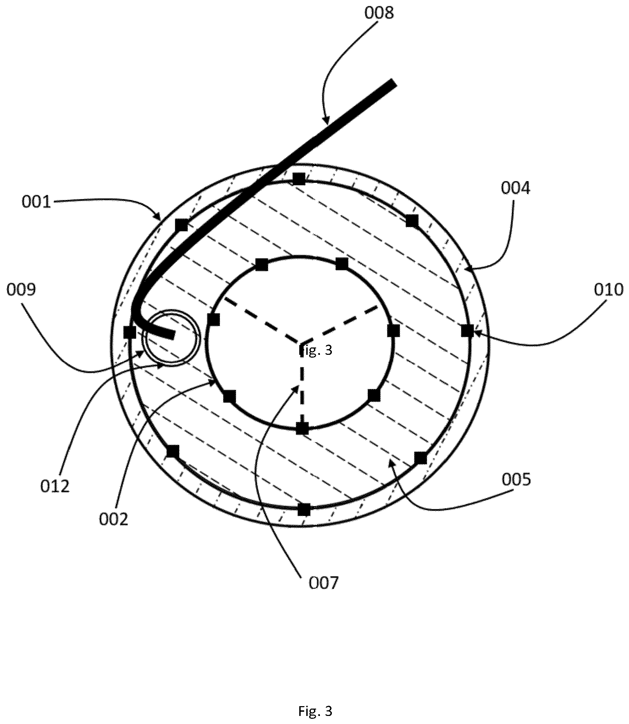

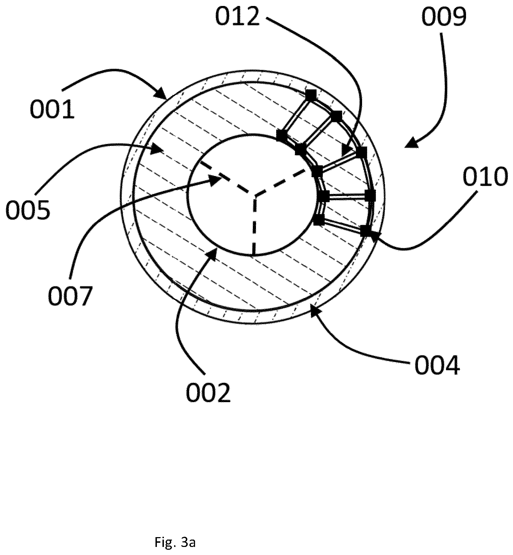

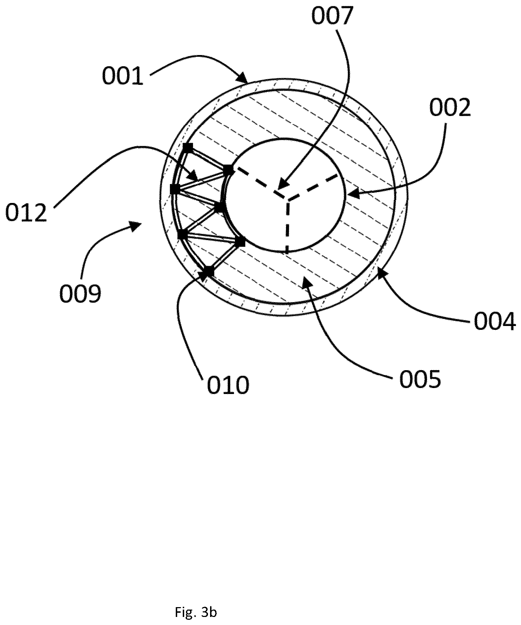

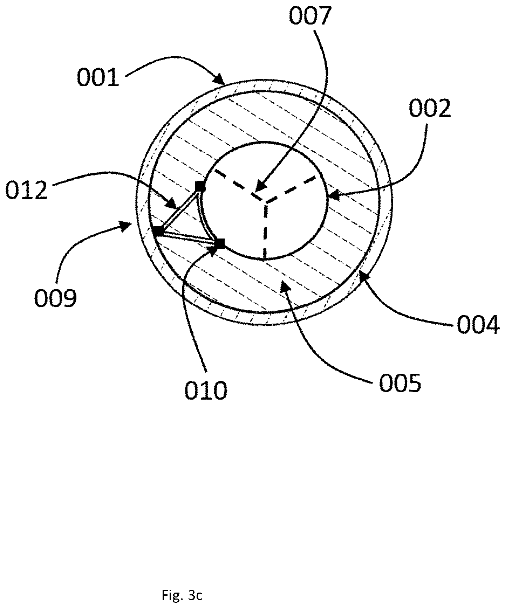

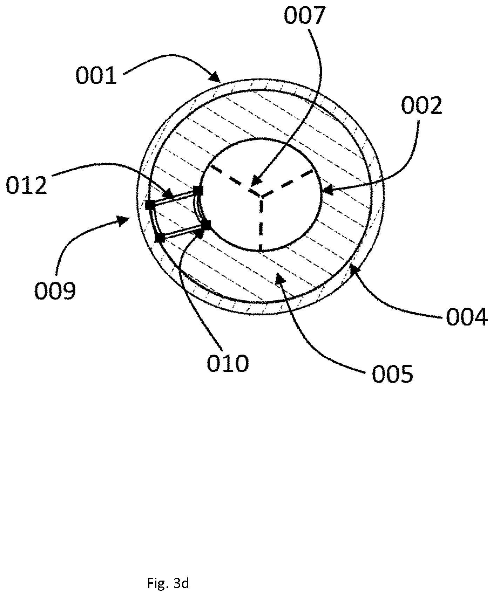

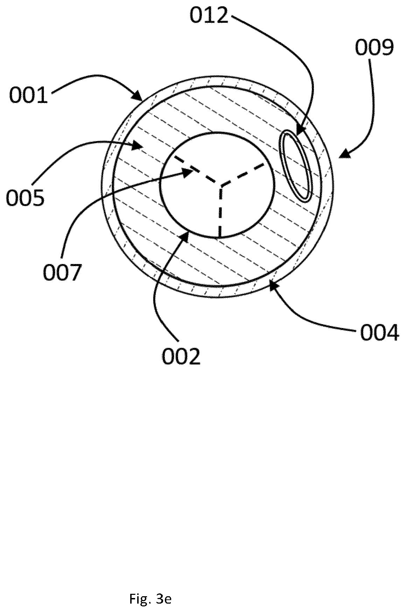

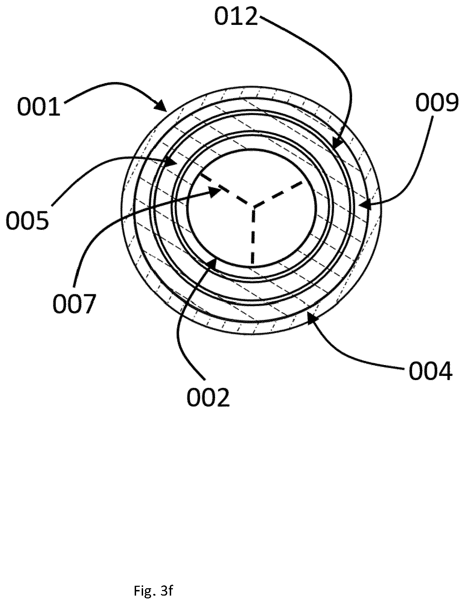

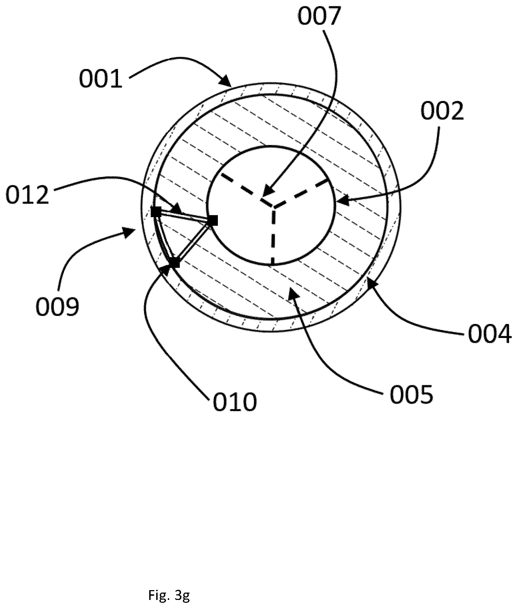

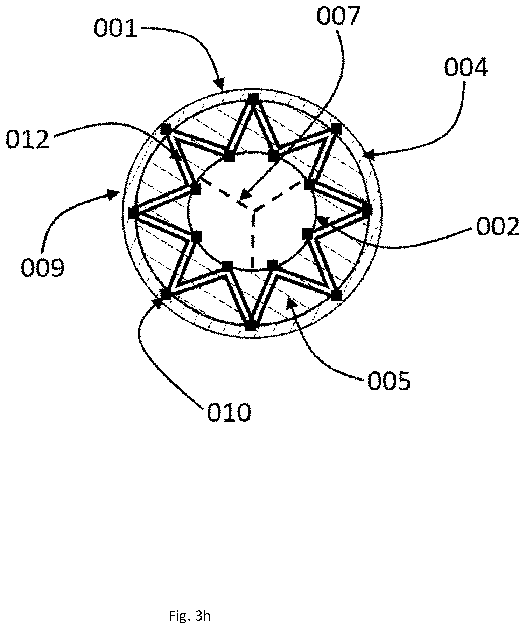

Fig 3a - 3h illustrate a number of possible designs for the lead gateway 009 wherein one or several lead gateways 009 may be included in the replacement heart valve prosthesis. The lead gateway 009 can be triangular, rectangular, quadrangular, or/and round, wherein the limit of the lead gateway 009 is characterized by a suture line 012, or the lead gateway 009 can be designed around the inner stent 002 (Fig. 3f). In Fig. 3f two concentric suture lines 012 are close to the inner stent 002 and close to the outer stent 001, respectively, wherein the probe or lead is positioned inbetween these suture lines 012 (not shown). The suture line 012 can by made by way of a suture within the sealing ring 005 wherein the suture line can be a single suture or double or multiple suture or a means forming an area within said means in order to move through and position a probe, a pacemaker lead or any other means without essentially interfering with the replacement heart valve functionality and avoiding essentially any leakage due to its positioning, providing stabilization of the probe or lead; allowing save and easy replacement when applicable, because of the predefined passage included in the prosthesis and thus no or only little ingrowth of endogenous tissue or structures and thus no or only reduced danger of damage of native structures occurs. The means can be made of a plastic, polymer, metal etc. and can be designed as a double clip being attached to each other from both sides of the sealing ring 005. In another aspect the lead gateway 009 can comprise a sealing or valve designed according to the need of the means to be positioned within the lead gateway 009. The lead gateway 009 can have varying diameters and dimensions, e.g. spanning the complete are between the outer stent 001 and inner stent 002 or only covering a part of the distance between the outer stent 001 and inner stent 002. In Fig. 3h several lead gateways 009 as described above are positioned around the circumference of the inner stent wherein at any position the lead or probe can be introduced and moved through the replacement heart valve stent prosthesis which allows for additional flexibility for the positioning of the probe or lead and thus does not restrict the operator or predetermine the position of the lead or probe which shall be positioned.

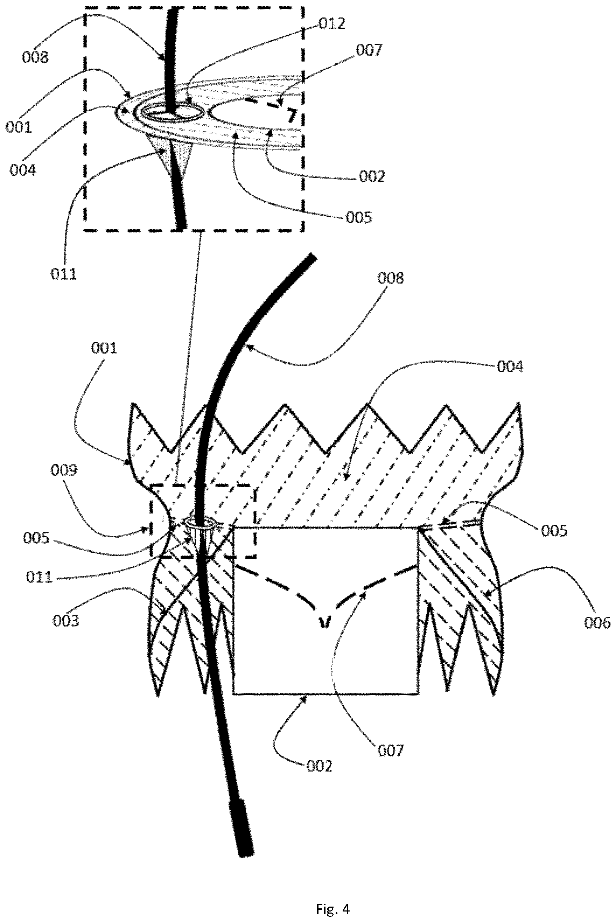

Fig. 4 shows a side view including a lead gateway 009 including a gateway homeostasis seal 011 positioned within the sealing ring 005 wherein a pacemaker lead 008 is positioned within the lead gateway 009. In the blow up depicted above the detail of the lead gateway 009 is illustrated wherein the lead gateway 009 is positioned within the sealing ring 005 between the outer stent 001 and the inner stent 002 including the heart valve 007 limited by the suture line 012 wherein the gateway hemostasis seal 011 is fixed within the lead gateway 009 forming a valve in direction to the ventricular part of the replacement heart valve prosthesis wherein the pacemaker lead 008 is positioned within the lead gateway 009 and wherein the gateway hemostasis seal 011 assures that essentially no leakage of blood during heart beat occurs.

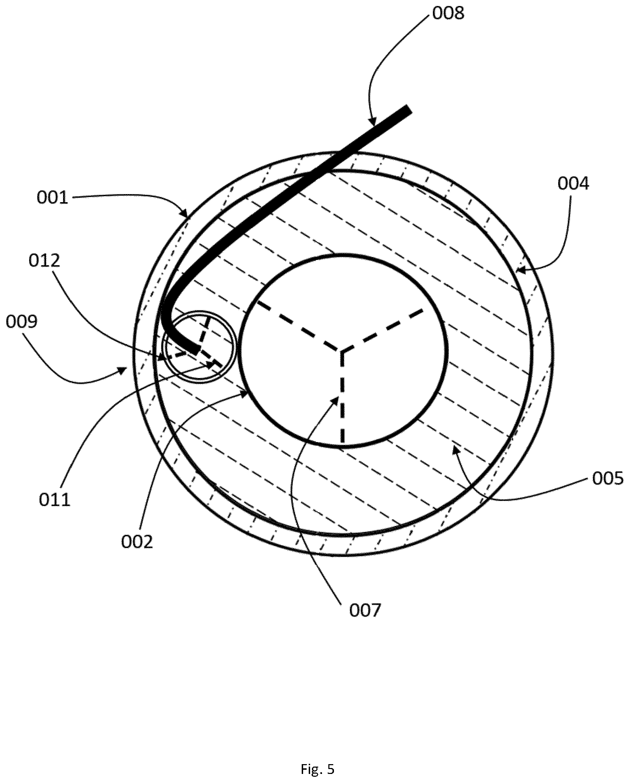

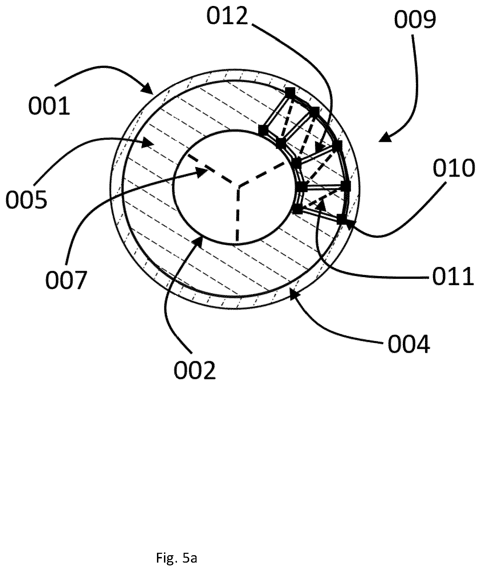

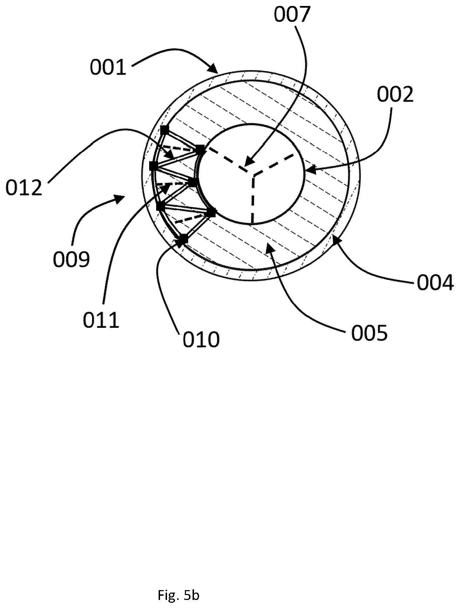

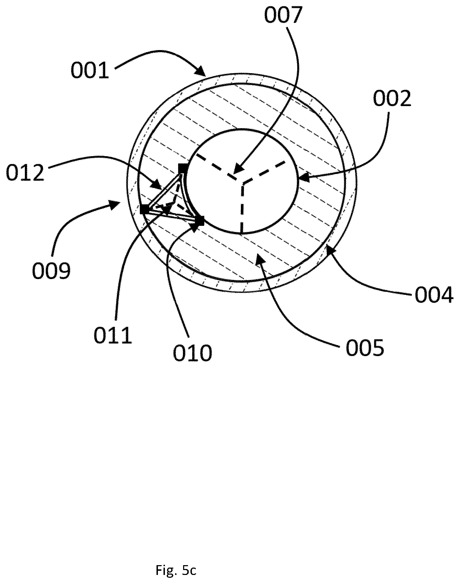

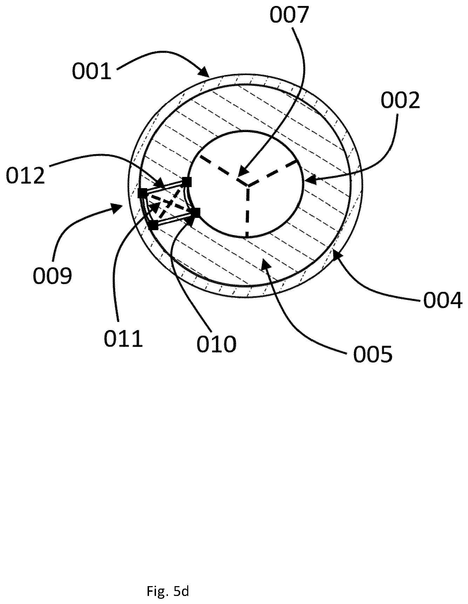

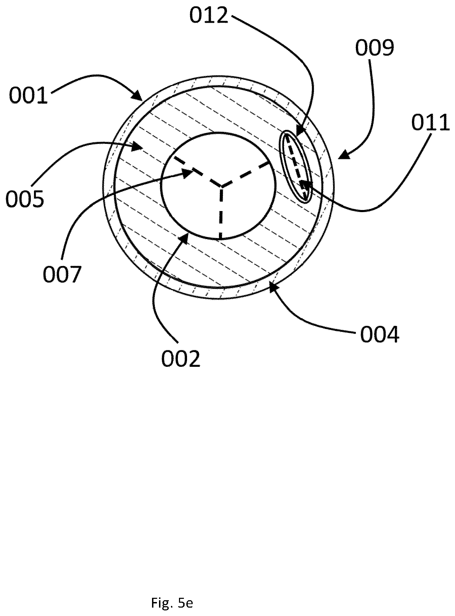

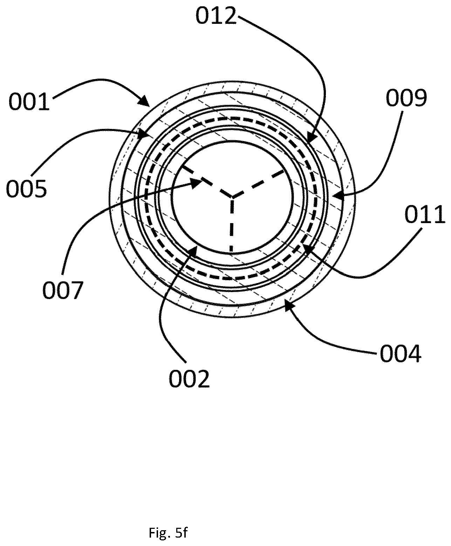

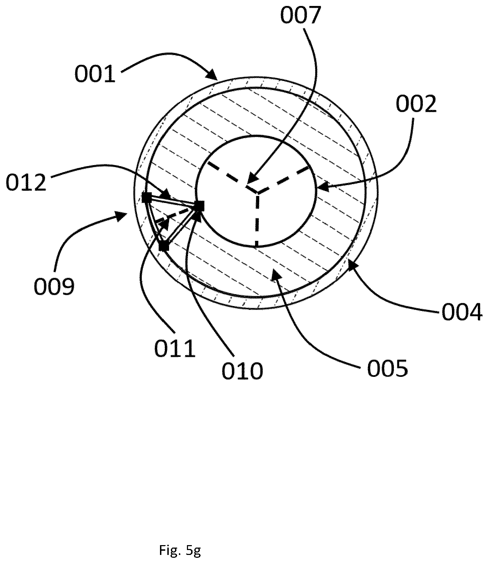

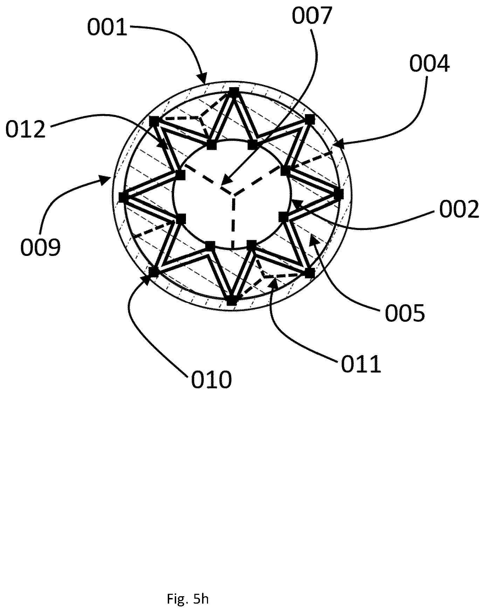

Fig. 5 is the same view as Fig. 3 wherein in lead gateway 009 a gateway hemostasis seal 011 is included.
Fig. 5a - 5h resemble Fig. 3a - 3h wherein the lead gateway 009 in each case includes a gateway hemostasis seal 011.

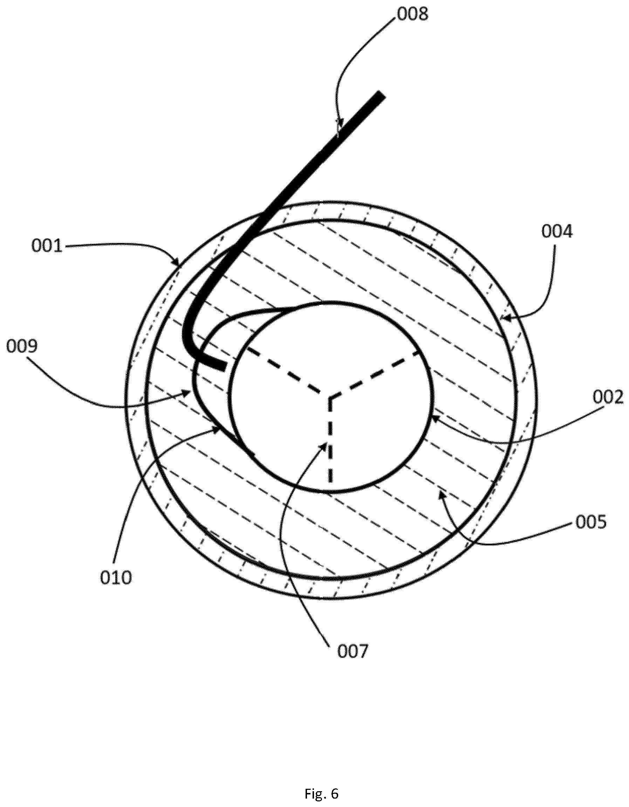

Fig. 6 illustrates a predefined passage integrated in the inner stent; The predefined passage 009 is positioned outside the inner stent and includes a hemostatic valve (not shown) wherein an accessory means like a lead or electrode can be passaged through and positioned for permanent positioning. It is also possible to have a single stent device including such a predefined passage integrated or aligned with the stent at the outside or inside of the stent.

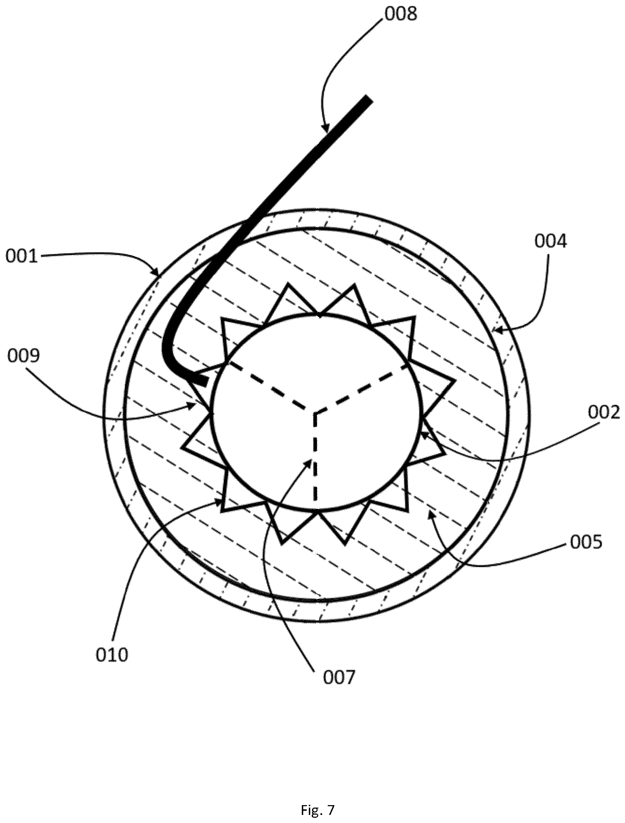

Fig. 7 illustrates an example of the disclosure wherein several predefined passages are foreseen at the outside to the inner stent. Same is possible in a single stent device.

### DETAILED DESCRIPTION OF THE DISCLOSURE

In the following certain terms will be defined in more detail, and it is to be understood that terms not defined herein shall be understood as is the common understanding of the applicable skilled person in the field.

A "replacement heart valve prosthesis" according to the disclosure shall be understood to relate to any medical device that can be delivered in a minimally invasive fashion or by way of a catheter based procedure. The terms can be used interchangeably. A prosthesis in the sense of the disclosure can be e.g. a stent or stent-based prosthesis or stent-based replacement heart valve prosthesis like an aortic replacement heart valve, a mitral replacement heart valve, a pulmonary replacement heart valve or a tricuspid replacement heart valve. It can be a single stent based device or a two part or three part or multiple part stent device optionally including connecting means.

A "connection means" or "connecting means" in the sense of the disclosure is a mechanical or physical connection of two struts in a stent or laser cut stent wherein two stents are connected to form a stable unit. The connecting means can be by e.g. welding, gluing or any other known procedure or process or means. A connecting means can also be an attachment or clipping means which exhibits a special design and geometry for releasable or nonreleasable connection in case a two-part or multiple part stent prosthesis is used.

A "sealing material" according to the disclosure shall be understood to relate to any natural or synthetic material, e.g. pericard of bovine or porcine origin, or synthetic material like any polymer.

A "predefined passage" or a "lead gateway" according to the disclosure shall be understood to relate to a position in the replacement heart valve prosthesis which is foreseen as a passage of an accessory means like a probe, a lead, an electrode, a catheter, etc. wherein a design is chosen and a means is included which essentially prevents unwanted leakage of blood during heart beat, optionally so that the accessory means can be passaged as required through the replacement heart valve prosthesis; one can chose to include and position as required and useful one, two, three, four, five or several predefined passages in the replacement heart valve prosthesis so that several of the same of different accessory means can be placed, positioned, fixed or/and removed in an easy and save manner. The accessory means can thus be positioned essentially without blood leakage and can in one aspect also be mechanically isolated from the remaining parts of the prosthesis avoiding interference with an essentially correct functionality of the prosthesis. A predefined passage in the sense of the disclosure can in one aspect be positioned on or in the sealing material outside the single stent, or in one aspect between the inner and outer stent or/and in one aspect essentially outside of the replacement heart valve, or in another aspect the predefined passage is aligned with the inside or outside of the single stent, or inside or outside of the inner stent or it forms an integral part of the single stent or of the inner stent. In another aspect the predefined passage is attached inside or outside of the single stent or inner stent, or the predefined passage is positioned with a distance from the inner stent of 1 to 5 mm or/and with a distance from the outer stent of 5 to 20 mm. In another aspect the inner stent or/and the predefined passage is mechanically isolated from the outer stent, or wherein the predefined passage is mechanically isolated from the single stent, the inner stent or/and the outer stent. Hence in one aspect a defined distance to the inner stent and to the outer stent can essentially avoid interference of the accessory means with either the inner stent or/and the outer stent. Hence the one or more predefined passage(s) allow for the access through a replacement heart valve prosthesis without jeopardizing its essentially leakage free function and give conveniently access to different parts or areas of the heart. This can be e.g. advantageous wherein access for a pacemaker or/and defibrillator is needed or wherein other electrodes or cables or probes for temporary or permanent implantation or/and investigational access is needed. This can concern the right or/and the left heart area, e.g. any area in the atrium or ventricle or tissue in between said areas or/and particular access to special areas for measuring or manipulation of the heart function.

An "accessory means" according to the disclosure shall be understood to relate to any means which is to be inserted temporarily or permanently into the heart of a patient which means needs to cross any of the endogenous valves and wherein such a endogenous valve needs to or has been replaced by a replacement heart valve prosthesis. Such an accessory means can be any selected from the group consisting of a probe, a catheter, a cable, an electrode, a lead, e.g. a pacemaker or defibrillator lead, and a camera.

A "suture line" according to the disclosure shall be understood to relate to the predefined passage containing a means which provides for a limitation for the accessory means, e.g. electrode, probe, lead, catheter, etc. and which aids or/and prevents damage to the sealing material surrounding the accessory means and predefined passage, optionally the suture line is radiopaque for better visualization during the procedure of introducing accessory means.

A "radiopaque marker" according to the disclosure shall be understood to relate to any suitable material by which one can visualize a point or area or e.g. a suture line by way of visualization means during an operation in a patient.

A "hemostatic valve" according to the disclosure shall be understood to relate to a sealing means which is capable to have any accessory means like a probe, lead, catheter, electrode, cable etc. introduced or moved through a predefined passage and which essentially prevents leakage of blood during heart beat wherein it may comprise 2, 3 or 4 leaflets or lips or tissue layers, optionally which is foldable or/and has a low profile.

"Mechanically isolated" according to the disclosure shall be understood to relate to an accessory means introduced within a replacement heart valve prosthesis and wherein the accessory means is positioned within or through said replacement heart valve prosthesis and which during heart beat does essentially not interfere with the heart beat, the replacement heart valve prosthesis and/or does not result in a blood leakage due to its positioning and the design of the predefined passage. Optionally special fixation means can be applied to maintain the accessory means in a predefined position to support that it essentially does not interfere with the functionality of the replacement heart valve prosthesis and heart beat.

"A horizontal or vertical movement during heart beating" according to the disclosure shall be understood to relate to the movement of the accessory means positioned within the replacement heart valve prosthesis.

"Essentially protected from a horizontal or vertical movement" according to the disclosure shall be understood to relate to the accessory means wherein due to the design of the lead gateway and optional means the movement of said accessory means is reduced to a minimum or functional minimum thus supporting essentially the functionality of the replacement heart valve prosthesis.

A "guidance means" according to the disclosure shall be understood as any means useful to help direct or adjust the accessory means in a defined position or direction in cooperation with the predefined passage, e.g. a means connected to the single stent or the inner stent or/and connected to the outer stent, which may be made of any useful material like nitinol, a natural or synthetic tissue.

The embodiments of the disclosure will be described in more detail in the following.

The object of the application is achieved in one aspect by a replacement heart valve prosthesis comprising a sealing material wherein the sealing material contains at least one predefined passage suitable for an accessory means, e.g. a catheter, a probe, an electrode or a lead or any other cable or means which needs to be placed into a heart of a patient temporarily or constantly.

The object of the application is achieved in another aspect the disclosure relates to a system comprising a replacement heart valve prosthesis as described herein and an accessory means, e.g. catheter, a probe, an electrode or a lead or any other cable or means which needs to be placed into a heart of a patient temporarily or constantly.

The object of the application is achieved in another aspect the disclosure relates to a method for introducingan accessory means, e.g. a probe or an electrode, in a patient carrying a replacement heart valve prosthesis as described herein wherein the predefined passage is visualized and the accessory means is introduced into and pushed through the predefined passage and positioned in its final target position.

The feature of a predefined passage for an accessory means like a catheter, a probe, an electrode or a lead provides for the possibility to introducing for implantation or investigational purposes certain means passing through the implanted replacement heart valve prosthesis without the need to perform surgery or interfere with a the sealing characteristics of the replacement heart valve prosthesis.

Moreover and in particular, it becomes now feasible to implant a pacemaker and/or a defibrillator and in particular the respective leads passing through the heart valve position without the risk of interference with a correct sealing of the replacement heart valve prosthesis.

Moreover, a certain patient group due to the embodiments according to the disclosure are now no more excluded from certain prosthesis implantations and can now be advantageously be treated with a prosthesis according to their medical needs.

It is also an advantage of the device according to the disclosure that it will be unproblematic to implant into a patient who has received a replacement heart valve prosthesis and which implantation lead to the need for the subsequent implantation of a pace maker or/and a defibrillator to implant such additional device(s) in an easy manner without the risk of interference with a correct functioning of the replacement heart valve prosthesis because the cables, leads or electrodes can be passed through the replacement heart valve prosthesis wherein one, two, three or several predefined passages are included. Thus interference by such an additional implant(s) with the replacement heart valve prosthesis is avoided. In certain circumstances a pacemaker or/and a defibrillator is implanted wherein these devices are positioned in the patient as is standard practice. The respective cables, electrodes and leads can be projected to any of the left or right heart ventricles and left and right atrium or in any tissue in between.

A further advantage of a predefined passage in a replacement heart valve prosthesis according to the disclosure is that the accessory means is held in a secure and predefined place, it does not move and not interfere with the functionality of the replacement heart valve prosthesis. Moreover, neither the replacement heart valve prosthesis nor any parts of the accessory means is prone to damage. Moreover electrical interference of any lead and the prosthesis is advantageously avoided. Also a change of a pacemaker or a defibrillator which may become necessary over the years can be performed without jeopardizing the replacement heart valve prosthesis during the procedure. In particular the position of the replacement heart valve prosthesis will remain the same and the danger of changing the position thereof is minimized or completely avoided. Finally the issue of paravalvular leakage in any circumstances is avoided.

In one further aspect the disclosure concerns a replacement heart valve as described herein wherein the replacement heart valve comprises at least an inner and an outer part, preferably an inner and an outer frame (or stent wherein in the following any suitable frame materials like metal, nitinol, plastic, rubber, composite is possible and the frame can be designed as suitable for the application or it can be a stent) or/and is a two-part stent or a one-part stent, and comprises a replacement heart valve.

In one further aspect the disclosure concerns a replacement heart valve prosthesis as described herein wherein the sealing material comprises a radiopaque suture line or/and a point or an area on or in the sealing material marked with a radiopaque marker.

In one further aspect the disclosure concerns a replacement heart valve prosthesis as described herein wherein the predefined passage contains a hemostatic valve, preferably wherein the hemostatic valve is positioned between or within one or more radiopaque suture lines or radiopaque markers.

In one further aspect the disclosure concerns a replacement heart valve prosthesis as described herein wherein the hemostatic valve comprises or is composed of natural or synthetic material, preferably any biocompatible material, e.g. pericard or/and a polymer.

In one further aspect the disclosure concerns a replacement heart valve prosthesis as described herein wherein the hemostatic valve comprises 2, 3 or 4 leaflets or lips or tissue layers.

In one further aspect the disclosure concerns a replacement heart valve prosthesis as described herein wherein the predefined passage or/and the radiopaque suture line or/and the point or the area on or in the sealing material marked with a radiopaque marker is positioned on or in the sealing material between the inner and outer part, preferably between the inner and outer stent, of the replacement heart valve prosthesis.

In one further aspect the disclosure concerns a replacement heart valve prosthesis as described herein wherein the predefined passage is aligned with the inner part or inner stent.

In one further aspect the disclosure concerns a replacement heart valve prosthesis as described herein wherein the predefined passage is positioned with a distance from the inner part or inner stent of 1 to 5 mm or/and with a distance from the outer part or stent of 5 to 20 mm.

In one further aspect the disclosure concerns a replacement heart valve prosthesis as described herein wherein the inner part or stent or/and the predefined passage is mechanically isolated from the outer part or stent.

In one further aspect the disclosure concerns a replacement heart valve prosthesis as described herein wherein an accessory means positioned within the predefined passage does essentially not effect a horizontal or vertical movement during heart beating or/and is essentially protected from a horizontal or vertical movement.

In one further aspect the disclosure concerns a replacement heart valve prosthesis as described herein wherein the hemostatic valve is foldable or/and has a low profile.

In one further aspect the disclosure concerns a replacement heart valve prosthesis as described herein wherein the replacement heart valve prosthesis comprises one or more guidance means for the accessory means.

In one further aspect the disclosure concerns a system as described herein wherein the accessory means like a pacemaker lead comprises one or more fixing means.

In one further aspect the disclosure concerns a suture line which is essentially cut proof, has low profile and is radiopaque.

In one further aspect the disclosure concerns a lead gateway including a hemostatic seal which has low profile and can be easily crimped together with the remaining replacement heart valve prosthesis to 14 to 40, or in particularly 14, 15, 16, 17, 18, 19, 20, 30, or 40 French.

In one further aspect the disclosure concerns a hemostatic seal having a variable diameter for convenient introduction of an accessory means like a probe, catheter, electrode, cable, lead etc.

### REFERENCE NUMBER LIST

- 001 -: Outer Stent
- 002 -: Inner Stent
- 003 -: Connecting Arm
- 004 -: Atrial Sealing
- 005 -: Sealing Ring
- 006 -: Ventricular Sealing
- 007 -: Prosthetic Valve
- 008 -: Accessory Means, here a pacemaker lead
- 009 -: Lead Gateway (predefined passage)
- 010 -: Frame Strut
- 011 -: Gateway Hemostasis Seal (seal in predefined passage)
- 012 -: Suture Line

## Claims

1. Replacement heart valve prosthesis comprising a sealing material wherein the sealing material contains at least one predefined passage suitable for an accessory means, e.g. a catheter, a probe or an electrode, and wherein the predefined passage contains a hemostatic valve.

2. Replacement heart valve prosthesis according to claim 1 wherein the replacement heart valve prosthesis comprises a frame, a replacement heart valve and sealing material, preferably a single stent or an inner and an outer frame, preferably an inner and outer stent .

3. Replacement heart valve prosthesis according to claim 1 or 2 wherein the sealing material comprises one or more radiopaque suture lines or/and one or more suture points or an area on or in the sealing material marked with one or more radiopaque markers.

4. Replacement heart valve prosthesis according to any of claims 1 to 3 wherein the hemostatic valve is positioned between or within one or more radiopaque suture lines or points or areas or radiopaque markers.

5. Replacement heart valve prosthesis according to any of claims 1 to 4 wherein the hemostatic valve comprises or is composed of natural or synthetic material, preferably any biocompatible material, e.g. pericard or/and one or more polymers.

6. Replacement heart valve prosthesis according to claim 5 wherein the hemostatic valve comprises 2, 3 or 4 leaflets or lips or tissue layers.

7. Replacement heart valve prosthesis according to any of the preceding claims wherein the predefined passage or/and the radiopaque suture line(s) or/and the point(s) or the area(s) on or in the sealing material marked with one or more radiopaque markers is positioned on or in the sealing material outside the single stent, or between the inner and outer frame or stent, or/and essentially outside of the replacement heart valve.

8. Replacement heart valve prosthesis according to claim 7 wherein the predefined passage is aligned with the inside or outside of the single stent, or inside or outside of the inner frame or stent or it forms an integral part of the single stent or of the inner frame or stent.

9. Replacement heart valve prosthesis according to claim 7 wherein the predefined passage is attached inside or outside of the single stent or inner frame or stent, or the predefined passage is positioned with a distance from the inner frame or stent of 1 to 5 mm or/and with a distance from the outer frame or stent of 5 to 20 mm.

10. Replacement heart valve prosthesis according to any of the preceding claims wherein the inner frame or stent or/and the predefined passage is mechanically isolated from the outer frame or stent, or wherein the predefined passage is mechanically isolated from the single stent, the inner frame or stent or/and the outer frame or stent.

11. Replacement heart valve prosthesis according to any of the preceding claims wherein the accessory means, e.g. a catheter, a probe or an electrode, positioned within the predefined passage does essentially not effect a horizontal or vertical movement during heart beating or/and is essentially protected or/and is essentially isolated from a horizontal or vertical movement.

12. Replacement heart valve prosthesis according to any of the preceding claims wherein the hemostatic valve is foldable or/and has a low profile.

13. Replacement heart valve prosthesis according to any of the preceding claims wherein the replacement heart valve prosthesis comprises one or more guidance means for the accessory means, e.g. a catheter, a probe or an electrode.

14. System comprising a replacement heart valve prosthesis according to any of claims 1 to 13 and the accessory means, e.g. a catheter, a probe or an electrode, preferably wherein the probe or lead, e.g. pacemaker or defibrillator lead, comprises one or more fixing means.

## Patentansprüche

1. Ersatzherzklappenprothese, die ein Dichtungsmaterial umfasst, wobei das Dichtungsmaterial mindestens einen vordefinierten Durchgang enthält, der für ein Zusatzmittel, z. B. einen Katheter, eine Sonde oder eine Elektrode, geeignet ist, wobei der vordefinierte Durchgang ein Hämostaseventil enthält.

2. Ersatzherzklappenprothese nach Anspruch 1, wobei die Ersatzherzklappenprothese umfasst: einen Rahmen, eine Ersatzherzklappe und Dichtungsmaterial, vorzugsweise einen einzelnen Stent oder einen inneren und einen äußeren Rahmen, vorzugsweise einen inneren und einen äußeren Stent.

3. Ersatzherzklappenprothese nach Anspruch 1 oder 2, wobei das Dichtungsmaterial eine oder mehrere röntgendichte Nahtlinien oder/und einen oder mehrere Nahtpunkte oder einen Bereich auf oder in dem Dichtungsmaterial umfasst, der mit einem oder mehreren röntgendichten Markern markiert ist.

4. Ersatzherzklappenprothese nach einem der Ansprüche 1 bis 3, wobei das Hämostaseventil zwischen oder innerhalb einer oder mehrerer röntgendichter Nahtlinien oder -punkte oder -bereiche oder röntgendichter Markierungen positioniert ist.

5. Ersatzherzklappenprothese nach einem der Ansprüche 1 bis 4, wobei das Hämostaseventil aus natürlichem oder synthetischem Material, vorzugsweise einem biokompatiblen Material, z. B. Perikard oder/und einem oder mehreren Polymeren, besteht oder zusammengesetzt ist.

6. Ersatzherzklappenprothese nach Anspruch 5, wobei das Hämostaseventil 2, 3 oder 4 Segel oder Lippen oder Gewebeschichten umfasst.

7. Ersatzherzklappenprothese nach einem der vorhergehenden Ansprüche, wobei der vordefinierte Durchgang oder/und die röntgendichte(n) Nahtlinie(n) oder/und der (die) Punkt(e) oder Bereich(e) auf oder in dem Dichtungsmaterial, der (die) mit einem oder mehreren röntgendichten Markern markiert ist (sind), auf oder in dem Dichtungsmaterial außerhalb des einzelnen Stents oder zwischen dem inneren und dem äußeren Rahmen oder Stent oder/und im Wesentlichen außerhalb der Ersatzherzklappenprothese positioniert sind.

8. Ersatzherzklappenprothese nach Anspruch 7, wobei der vordefinierte Durchgang mit der Innenseite oder Außenseite des einzelnen Stents oder der Innenseite oder Außenseite des inneren Rahmens oder Stents ausgerichtet ist oder einen integralen Bestandteil des einzelnen Stents oder des inneren Rahmens oder Stents bildet.

9. Ersatzherzklappenprothese nach Anspruch 7, wobei der vordefinierte Durchgang innerhalb oder außerhalb des einzelnen Stents oder inneren Rahmens oder Stents angebracht ist oder der vordefinierte Durchgang in einem Abstand von 1 bis 5 mm vom inneren Rahmen oder Stent oder/und in einem Abstand von 5 bis 20 mm vom äußeren Rahmen oder Stent positioniert ist.

10. Ersatzherzklappenprothese nach einem der vorhergehenden Ansprüche, wobei der innere Rahmen oder Stent oder/und der vordefinierte Durchgang mechanisch vom äußeren Rahmen oder Stent isoliert ist oder wobei der vordefinierte Durchgang mechanisch vom einzelnen Stent, dem inneren Rahmen oder Stent oder/und dem äußeren Rahmen oder Stent isoliert ist.

11. Ersatzherzklappenprothese nach einem der vorhergehenden Ansprüche, wobei das Zusatzmittel, z. B. ein Katheter, eine Sonde oder eine Elektrode, das innerhalb des vordefinierten Durchgangs positioniert ist, während des Herzschlags im Wesentlichen keine horizontale oder vertikale Bewegung ausführt oder/und gegen eine horizontale oder vertikale Bewegung im Wesentlichen geschützt ist oder/und im Wesentlichen isoliert ist.

12. Ersatzherzklappenprothese nach einem der vorhergehenden Ansprüche, wobei das Hämostaseventil faltbar ist oder/und ein flaches Profil aufweist.

13. Ersatzherzklappenprothese nach einem der vorhergehenden Ansprüche, wobei die Herzklappenprothese eine oder mehrere Führungseinrichtungen für das Zusatzmittel, z. B. einen Katheter, eine Sonde oder eine Elektrode, umfasst.

14. System, umfassend eine Herzklappenprothese nach einem der Ansprüche 1 bis 13 und das Zusatzmittel, z. B. einen Katheter, eine Sonde oder eine Elektrode, vorzugsweise wobei die Sonde oder Leitung, z. B. eine Herzschrittmacher- oder Defibrillatorleitung, ein oder mehrere Befestigungsmittel umfasst.

## Revendications

1. Prothèse de valvule cardiaque de remplacement comprenant un matériau d'étanchéité, le matériau d'étanchéité contenant au moins un passage prédéfini adapté à un moyen accessoire, par exemple un cathéter, une sonde ou une électrode, et le passage prédéfini contenant une valve hémostatique.

2. Prothèse de valvule cardiaque de remplacement selon la revendication 1, ladite prothèse de valvule cardiaque de remplacement comprenant un cadre, une valvule cardiaque de remplacement et un matériau d'étanchéité, de préférence un stent unique ou un cadre interne et un cadre externe, de préférence un stent interne et un stent externe.

3. Prothèse de valvule cardiaque de remplacement selon la revendication 1 ou 2, dans laquelle le matériau d'étanchéité comprend une ou plusieurs lignes de suture radio-opaques ou/et un ou plusieurs points de suture ou une zone sur ou dans le matériau d'étanchéité marquée par un ou plusieurs marqueurs radio-opaques.

4. Prothèse de valvule cardiaque de remplacement selon l'une quelconque des revendications 1 à 3, dans laquelle la valve hémostatique est positionnée entre ou à l'intérieur d'une ou plusieurs lignes de suture radio-opaques ou points ou zones ou marqueurs radio-opaques.

5. Prothèse de valvule cardiaque de remplacement selon l'une quelconque des revendications 1 à 4, dans laquelle la valve hémostatique comprend ou est composée d'un matériau naturel ou synthétique, de préférence tout matériau biocompatible, par exemple du péricarde ou/et un ou plusieurs polymères.

6. Prothèse de valvule cardiaque de remplacement selon la revendication 5, dans laquelle la valve hémostatique comprend 2, 3 ou 4 feuillets ou lèvres ou couches de tissu.

7. Prothèse de valvule cardiaque de remplacement selon l'une quelconque des revendications précédentes, dans laquelle le passage prédéfini ou/et la ou les lignes de suture radio-opaques ou/et le ou les points ou la ou les zones sur ou dans le matériau d'étanchéité marqués par un ou plusieurs marqueurs radio-opaques sont positionnés sur ou dans le matériau d'étanchéité à l'extérieur du stent unique, ou entre le cadre ou stent intérieur et extérieur, ou/et essentiellement à l'extérieur de la valvule cardiaque de remplacement.

8. Prothèse de valvule cardiaque de remplacement selon la revendication 7, dans laquelle le passage prédéfini est aligné avec l'intérieur ou l'extérieur du stent unique, ou l'intérieur ou l'extérieur du cadre ou stent interne, ou fait partie intégrante du stent unique ou du cadre ou stent interne.

9. Prothèse de valvule cardiaque de remplacement selon la revendication 7, dans laquelle le passage prédéfini est fixé à l'intérieur ou à l'extérieur du stent unique ou du cadre ou stent interne, ou le passage prédéfini est positionné à une distance comprise entre 1 et 5 mm du cadre ou stent interne ou/et à une distance comprise entre 5 et 20 mm du cadre ou stent externe.

10. Prothèse de valvule cardiaque de remplacement selon l'une quelconque des revendications précédentes, dans laquelle le cadre ou stent interne ou/et le passage prédéfini sont isolés mécaniquement du cadre ou stent externe, ou dans laquelle le passage prédéfini est isolé mécaniquement du stent unique, du cadre ou stent interne ou/et du cadre ou stent externe.

11. Prothèse de valvule cardiaque de remplacement selon l'une quelconque des revendications précédentes, dans laquelle le moyen accessoire, par exemple un cathéter, une sonde ou une électrode, positionné à l'intérieur du passage prédéfini, n'effectue essentiellement aucun mouvement horizontal ou vertical pendant les battements cardiaques ou/et est essentiellement protégé ou/et est essentiellement isolé d'un mouvement horizontal ou vertical.

12. Prothèse de valvule cardiaque de remplacement selon l'une quelconque des revendications précédentes, dans laquelle la valve hémostatique est pliable ou/et présente un profil bas.

13. Prothèse de valvule cardiaque de remplacement selon l'une quelconque des revendications précédentes, la prothèse de valvule cardiaque de remplacement comprenant un ou plusieurs moyens de guidage pour le moyen accessoire, par exemple un cathéter, une sonde ou une électrode.

14. Système comprenant une prothèse de valvule cardiaque de remplacement selon l'une quelconque des revendications 1 à 13 et le moyen accessoire, par exemple un cathéter, une sonde ou une électrode, de préférence dans lequel la sonde ou le fil, par exemple un fil de stimulateur cardiaque ou de défibrillateur, comprend un ou plusieurs moyens de fixation.
